(19) Europäisches Patentamt
European Patent Office
Office européen des brevets

(11) **EP 2 029 520 B1**

(12) **EUROPÄISCHE PATENTSCHRIFT**

(45) Veröffentlichungstag und Bekanntmachung des Hinweises auf die Patenterteilung:
**31.07.2013   Patentblatt 2013/31**

(21) Anmeldenummer: **07704345.3**

(22) Anmeldetag: **05.02.2007**

(51) Int Cl.:
*C07C 209/16* (2006.01)       *C07C 211/10* (2006.01)
*C07C 211/14* (2006.01)       *C07C 213/02* (2006.01)
*C07C 215/08* (2006.01)

(86) Internationale Anmeldenummer:
**PCT/EP2007/051061**

(87) Internationale Veröffentlichungsnummer:
**WO 2007/093514 (23.08.2007 Gazette 2007/34)**

(54) **VERFAHREN ZUR HERSTELLUNG VON ETHYLENAMINEN UND ETHANOLAMINEN AUS MONOETHYLENGLYKOL (MEG)**

METHOD FOR PRODUCING ETHYLENE AMINES AND ETHANOL AMINES FROM MONOETHYLENE GLYCOL (MEG)

PROCÉDÉ POUR PRODUIRE DES ÉTHYLÈNEAMINES ET DES ÉTHANOLAMINES À PARTIR DE MONOÉTHYLÈNEGLYCOL (MEG)

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.02.2006   EP 06101643**

(43) Veröffentlichungstag der Anmeldung:
**04.03.2009   Patentblatt 2009/10**

(73) Patentinhaber: **BASF SE**
**67056 Ludwigshafen (DE)**

(72) Erfinder:
• **CAUWENBERGE, Gunther van**
**B-9140 Temse (BE)**
• **MELDER, Johann-Peter**
**67459 Böhl-Iggelheim (DE)**

• **HOFFER, Bram Willem**
**69120 Heidelberg (DE)**
• **KRUG, Thomas**
**67550 Worms (DE)**
• **PICKENÄCKER, Karin**
**68623 Lampertheim (DE)**
• **PAPE, Frank-Friedrich**
**67259 Kleinniedesheim (DE)**
• **SCHWAB, Ekkehard**
**67434 Neustadt (DE)**

(56) Entgegenhaltungen:
**EP-A- 0 839 575     US-A- 4 855 505**

Bemerkungen:
Die Akte enthält technische Angaben, die nach dem Eingang der Anmeldung eingereicht wurden und die nicht in dieser Patentschrift enthalten sind.

**EP 2 029 520 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung von Ethylenaminen und Ethanolaminen durch hydrierende Aminierung von Monoethylenglykol (im Folgenden: MEG) und Ammoniak in Gegenwart eines Katalysators.

In bekannten Verfahren wird in der Regel ein Gemisch an Ethanolaminen und Ethylenaminen erhalten; hiervon sind insbesondere Ethylendiamin (im Folgenden: EDA) und Diethylentriamin (Bis(2-aminoethyl)amin; im Folgenden: DETA), wichtige Wertstoffe, unter anderem zur Verwendung als Lösungsmittel, Stabilisatoren, zur Synthese von Chelat-Bildnern, Kunstharzen, Arzneimitteln, Inhibitoren und grenzflächenaktiven Substanzen.

EDA wird insbesondere als Rohstoff für Fungizide und Insektizide eingesetzt.

DETA findet insbesondere Verwendung als Lösungsmittel für Farbstoffe und ist Ausgangsmaterial zur Herstellung von Ionenaustauschern, Schädlingsbekämpfungsmitteln, Antioxidantien, Korrosionsschutzmitteln, Komplexbildnern, Textilhilfsmitteln und Absorptionsmitteln für (saure) Gase.

Nicht-lineare Amine im Produktgemisch der Ethylenamine und Ethanolamine und insbesondere zyklische Amine, überwiegend Piperazin und Piperazinderivate, sind dagegen weniger begehrt bis unerwünscht.

Zur Herstellung von Ethylenaminen sind in der Literatur zahlreiche Verfahren beschrieben.

Gemäß PEP Report No. 138, "Alkyl Amines", SRI International, 03/1981, insbesondere Seiten 7, 8 13-16, 43-107, 113, 117, liefert die Umsetzung von Dichlorethan mit Ammoniak bei Molverhältnissen von 1:15 DETA mit einem Anteil an den gebildeten Ethylenaminen von größer 20 Gew.-%. Neben 40 Gew.-% EDA fallen jedoch 40 Gew.-% höhere Ethylenamine an.

Durch Aminierung von Monoethanolamin (im Folgenden: MEOA) mit Ammoniak (vgl. zum Beispiel den oben genannten PEP Report, US 4,014,933 (BASF AG)) kann die Bildung dieser höheren Ethylenamine (d. h. Ethylenaminen mit einem Siedepunkt über dem von Triethylentetramin (im Folgenden: TETA)) zugunsten von EDA weitgehend zurückgedrängt werden. Als Nebenprodukte fallen jedoch bei dieser Umsetzung Aminoethylethanolamin (im Folgenden: AEEA) und Piperazin (im Folgenden: PIP) an.

Ind. Eng. Chem. Prod. Res. Dev. 1981, 20, Seiten 399-407, (C.M. Barnes et al.) beschreibt die Ammonolyse von MEOA zu EDA an Nickel-Katalysatoren auf einem $SiO_2$-$Al_2O_3$-Mischträger. Zusatz von Wasser und der gepulverte Katalysator waren angeblich vorteilhaft bei der Erhöhung der Ausbeute an EDA.

Nachteile dieser Technologien ergeben sich bei der Suspensionskatalyse u. a. aus der notwendigen Katalysator/Produkt Abtrennung. Darüber hinaus sind die Selektivitäten, z. B. für die Bildung von DETA, verbesserungswürdig.

WO-A-05/014623 (BASF AG) betrifft ein Verfahren zur Herstellung von Ethylenaminen (z. B. DETA) durch Umsetzung von MEOA mit Ammoniak in Gegenwart eines Katalysators in einem Reaktor (1) und Auftrennung des resultierenden Reaktionsaustrags, wobei bei der Auftrennung erhaltenes EDA in einem separaten Reaktor (2) in Gegenwart eines Katalysators zu DETA umgesetzt und der resultierende Reaktionsaustrag der Auftrennung des aus Reaktor 1 resultierenden Reaktionsaustrags zugeführt wird.

Die ältere deutsche Patentanmeldung Nr. 102005019373.0 vom 26.04.05 (BASF AG) betrifft ein Verfahren zur Herstellung von Ethylenaminen, in dem man in einer ersten Reaktionsstufe Ethylenoxid mit Ammoniak unter wasserfreien Bedingungen an einem anorganischen Ionenaustauscher kontinuierlich umsetzt, wobei das resultierende Umsetzungsprodukt Monoethanolamin, Diethanolamin und Triethanolamin in einem bestimmten Gewichtsverhältnis enthält, und das Umsetzungsprodukt anschließend kontinuierlich in einer zweiten Reaktionsstufe mit Ammoniak in Gegenwart von Wasserstoff und einem Hydrierkatalysator umsetzt.

Das Verfahren hat jedoch insbesondere den Nachteil, dass von Ethylenoxid hoher Reinheit ausgegangen werden muss.

Aus der DE 102005047464.0 ist es bekannt, die nicht zyklischen Amine, insbesondere EDA und DETA in hohen Ausbeuten und Selektivitäten durch hydrierende Aminierung ausgehend von MEOA zu erhalten, indem ein Heterogenkatalysator, enthaltend Verbindungen des Aluminiums, Kupfers, Nickels und Kobalts, eingesetzt wird, aufweisend eine Geometrie der Katalysatorformkörper entsprechend der nachfolgenden Definition:

Die Katalysatorformkörper sollen bei Kugel- oder Strangform jeweils einen Durchmesser von < 3 mm, bei Tablettenform eine Höhe von < 3 mm und bei allen anderen Geometrien jeweils einen Äquivalentdurchmesser L=1/a' von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorpartikels ($mm_s^2$) und $V_p$ das Volumen des Katalysatorpartikels ($mm_p^3$) ist.

Aus der WO 96/38226 sind Katalysatoren bekannt, enthaltend Rhenium, Nickel, Kobalt, Bor und Kupfer und/oder Ruthenium auf einem Trägermaterial, bei einem Gewichtsverhältnis von Nickel zu Rhenium im Bereich von 1 bis 30 und einem Gewichtsverhältnis von Nickel zu Kobalt, von Nickel zu Bor sowie von Nickel zu Kupfer und/oder Ruthenium von jeweils 1 zu 20. Der Katalysator wird zur reduktiven Aminierung von Alkanen oder Arylalkanderivaten, beispielsweise mit Ammoniak in Gegenwart von Wasserstoff, insbesondere von MEOA eingesetzt. Beispielhaft angegeben ist auch eine Vielzahl weiterer Alkanderivate als Edukte, darunter Ethylenglykol. Aus dem Dokument ist nicht zu entnehmen, dass der beschriebene Katalysator für den Verfahrensschritt der Umsetzung von Monoethylenglykol zu Monoethanolamin eine erhöhte Selektivität gegenüber den Folgereaktionen von Monoethanolamin aufweisen würde.

Aus US 4,855,505 ist ein Verfahren zur Hydroaminierung von beispielsweise Monoethylenglykol mit beispielsweise Ammoniak in Gegenwart eines Katalysators bekannt, der 4 bis 40 Gew.-% Nickel oder Kobalt und 0,1 bis 5 Gew.-% Ruthenium, das als Lösung eines Rutheniumhalogenids eingeführt wurde, auf einem porösen Metalloxidträger, enthaltend mindestens 50 Gew.-% aktiviertes Aluminiumoxid, enthält. Der Katalysator wird beispielhaft in Form von Tabletten mit einer Länge und einem Durchmesser von etwa 3 mm eingesetzt. In dem Dokument ist deutlich herausgestellt, dass Nichthalogenide, wie Rutheniumoxid, Rutheniumsulfat, Rutheniumnitrat, Rutheniumnitrosylnitrat, Rutheniumammoniumnitrat, Rutheniumacetylacetonat und Kaliumperruthenat, verstärken zwar die Aktivität eines Nickel- oder Kobaltkatalysators, ohne jedoch eine nennenswerte Verbesserung der Selektivität in organischen Hyderierungen im Vergleich zu den entsprechenden Katalysatoren ohne Zusatz von Ruthenium, zu bewirken. In Journal of Catalysis 161, Seiten 96 bis 106 (1996) ist der Einsatz von geträgerten sowie ungeträgerten Ruthenium-Kobalt-Katalysatoren für Hydrierungen, insbesondere von Nitrilverbindungen, beschrieben. Das Dokument gibt jedoch keinen Hinweis auf den Einsatz von Katalysatoren, enthaltend Ruthenium und Kobalt für die hydrierende Aminierung von Monoethylenglykol mit Ammoniak.

Aus der EP-A 0 839 575 ist ein Aminierungskatalysator bekannt, umfassend, bezogen auf das Gesamtgewicht des Katalysators mehr als 6 bis 50 Gew.-% Kobalt, Nickel oder deren Gemisch, 0,001 bis 25 Gew.-% Ruthenium, 0 bis 10 Gew.-% Kupfer und 0 bis 5'Gew.-% Promotoren auf einem porösen Metalloxidträger, wobei der Träger nicht mit Halogenverbindungen imprägniert wird. Die Katalysatoren verursachen insbesondere keine Korrosion bei gleichzeitig guter Haltbarkeit und hoher Selektivität in Bezug auf Ethylendiamin bei der Aminierung von Monoethanolamin mit Ammoniak.

Es war demgegenüber Aufgabe der Erfindung, ein Verfahren zur Herstellung von Ethylenaminen und Ethanolaminen durch hydrierende Aminierung von MEG mit erhöhter Selektivität für lineare Ethylenamine und Ethanolamine zur Verfügung zu stellen.

Die Lösung besteht in einem Verfahren zur Herstellung von Ethylenaminen und Ethanolminen durch hydrierende Aminierung von Monoethylenglykol und Ammoniak in Gegenwart eines Katalysators, das dadurch gekennzeichnet ist, dass das Verfahren in zwei Verfahrensstufen durchgeführt wird, wobei

- in der ersten Verfahrensstufe die Aminierung an einem Hydroaminierungskatalysator bis zu einem Monoethylenglykol-Umsatz von maximal 40 % durchgeführt wird und
- in der zweiten Verfahrensstufe ein geträgerter Katalysator mit einer Aktivmasse enthaltend Ruthenium und Kobalt und daneben kein weiteres Metall der Gruppe VIII sowie kein Metall der Gruppe IB, eingesetzt wird, in Form von Katalysatorformkörpern, die bei Kugelform oder Strangform jeweils einen Durchmesser von < 3 mm, bei Tablettenform eine Höhe von < 3 mm und bei allen anderen Geometrien jeweils einen Äquivalentdurchmesser $L = 1/a'$ von < 0,70 mm aufweisen, wobei $a'$ die externe Oberfläche per Volumeneinheit ($mm_s^2/mm_p^3$) ist, mit:

$$a' = \frac{Ap}{Vp}$$

wobei $A_p$ die externe Oberfläche des Katalysatorformkörpers ($mm_s2$) und $V_p$ das Volumen des Katalysatorformkörpers ($mm_p^3$) ist, eingesetzt wird und die zweite Verfahrensstufe bei einer um mindestens 10 °C höheren Temperatur als die erste Verfahrensstufe durchgeführt wird

Bevorzugt wird die erste Verfahrensstufe bis zu einem Monoethylenglykol-Umsatz von maximal 30 % durchgeführt.

Die Oberfläche und das Volumen des Katalysatorformkörpers ergeben sich aus den geometrischen Abmessungen des Formkörpers gemäß den bekannten mathematischen Formeln.

Das Volumen kann auch nach folgender Methode berechnet werden, bei der man:

1. die innere Porosität des Formkörpers bestimmt (z. B. über Messung der Wasseraufnahme in [ml/g Kat] bei Raumtemperatur und 1 bar Gesamtdruck),
2. die Verdrängung des Formkörpers beim Eintauchen in eine Flüssigkeit (z. B. durch Gasverdrängung mittels Helium-Pyknometer) bestimmt und
3. die Summe beider Volumina bildet.

Die Oberfläche kann auch nach folgender Methode theoretisch berechnet werden, bei der man eine Umhüllende des Formkörpers definiert, deren Kurvenradien max. 5 μm beträgt (um nicht die innere Porenoberfläche durch "Eindringen" der Umhüllenden in die Poren mitzunehmen) und die den Formkörper möglichst innig berührt (keine Schnittfläche mit dem Träger). Anschaulich würde das einer sehr dünnen Folie entsprechen, die man um den Formkörper legt und dann von innen ein Vakuum anlegt, so dass sich die Folie möglichst eng um den Formkörper legt.

Das als Edukt eingesetzte MEG kann nach bekannten Verfahren, beispielsweise durch Umsetzung von Ethylenoxid mit Wasser, festgestellt werden.

Die erfindungsgemäße Umsetzung erfolgt im Allgemeinen in der ersten Verfahrensstufe bei einer Temperatur im Bereich von 130 bis 230°C, bevorzugt von 150 bis 170°C, auch in der zweiten Verfahrensstufe bei einer Temperatur im Bereich von 150 bis 240°C, bevorzugt zwischen 160 und 190°C, wobei die Temperatur in der zweiten Verfahrensstufe stets um mindestens 10°C höher sein muss gegenüber der Temperatur in der ersten Verfahrensstufe. Die erfindungsgemäße Umsetzung erfolgt im Allgemeinen bei einem Absolutdruck im Bereich von 1-250 bar, bevorzugt 100-200 bar, insbesondere bei 150-200 bar.

MEG und Ammoniak werden in der ersten Verfahrensstufe bevorzugt in einem Molverhältnis im Bereich von 3 bis 30, bevorzugt von 7 bis 15, eingesetzt.

Die Erfindung ist nicht eingeschränkt bezüglich der in der ersten Verfahrensstufe eingesetzten Katalysatoren; es können alle bekannten Hydroaminierungskatalysatoren eingesetzt werden, beispielsweise Katalysatoren, enthaltend Nickel, Kobalt und Kupfer als Aktivmasse. Wesentlich ist, dass die Umsetzung in der ersten Verfahrensstufe auf maximal 40 % Monoethylenglykol, bevorzugt auf maximal 30 %, begrenzt wird.

Dabei wird ein Gemisch erhalten, in dem MEOA überwiegt, und das unmittelbar, insbesondere ohne Zwischenabtrennung von Ammoniak, in die zweite Verfahrensstufe eingeleitet wird.

In der zweiten Verfahrensstufe werden im Allgemeinen geträgerte Katalysatoren in einer Form eingesetzt, die nur aus der katalytisch wirksamen Aktivmasse im gegebenenfalls einem Verformungshilfsmittel, beispielsweise Graphit oder Stearinsäure besteht, oder als geträgerter Katalysator, d. h. die katalytisch wirksame Aktivmasse ist auf einen weitgehend inaktiven Träger aufgebracht. Bei Einsatz von geträgerten Katalysatoren beträgt der Anteil des Trägers an der Gesamtmasse des Katalysators (Aktivmasse plus Träger) bevorzugt 10 bis 90 Gew.-%.

Als Träger können alle bekannten geeigneten Träger verwendet werden, beispielsweise Aktivkohle, Siliciumcarbid oder Metalloxide. Der Einsatz von Metalloxiden ist bevorzugt. Von den Metalloxiden werden vorzugsweise Aluminiumoxid, Siliciumdioxid, Titandioxid, Zirkoniumdioxid, Ceriumdioxid, Zinkoxid, Magnesiumoxid oder deren Gemische verwendet, die gegebenenfalls mit Alkali- und/oder Erdalkalimetalloxiden dotiert sind. Besonders bevorzugt sind γ-Aluminiumoxid, Siliciumdioxid, Zirkoniumdioxid, Ceriumdioxid oder Titandioxid oder Gemische davon.

Bevorzugt ist der Träger γ-Aluminiumoxid.

Die Träger können in beliebiger Form eingesetzt werden, beispielsweise als Extrudate (in Form von Strängen), Pellets, Tabletten, Monolithe, Gewebe, Gestricke oder pulverförmig.

Die geträgerten Katalysatoren können nach allgemein bekannten Verfahren hergestellt werden. Hierzu zählt etwa das Tränken eines Trägers mit Lösungen von Verbindungen der eingesetzten Metallkomponenten. Als Lösungsmittel eignen sich alle üblichen Lösungsmittel, etwa Wasser, Methanol, Ethanol oder Aceton, vorzugsweise wird Wasser eingesetzt. Weiterhin kann der Katalysator durch gemeinsames oder sequentielles Fällen der Katalysatorkomponenten, anschließende Filtration und Waschen des Filterkuchens hergestellt werden. An das Tränken bzw. Fällen schließen sich ein Trocknungsschritt (50 bis 200°C) und ein Calcinationsschritt (200 bis 500°C) an. Die Katalysatoren werden dann reduziert bei Endtemperaturen von 200 bis 400°C und können anschließen passiviert werden, da die reduzierten Metalle pyrophor sind. Nach Einbau der Katalysatoren in den Synthesereaktor können die Katalysatoren vor Anfahren der Reaktion durch Reduktion mit Wasserstoff bei Temperaturen zwischen 100 und 300°C reaktiviert werden.

Der Katalysator enthält als Aktivmasse Ruthenium und Kobalt in elementarer Form und daneben kein weiteres Metall der Gruppe VIII sowie kein Metall der Gruppe IB.

Zur Herstellung des Katalysators können Verbindungen der genannten Metalle eingesetzt werden, bevorzugt Oxide, aber auch Nitrate, Carbonate oder Acetate. Die Metallverbindungen werden zur Fertigstellung des Katalysators vor Einsatz desselben in der hydrierenden Aminierung reduziert, vorzugsweise durch Behandeln mit Wasserstoff. So entsteht ein Katalysator, der die genannten Metalle in elementarer, fein verteilter Form, enthält. Hierbei beträgt der Anteil des Katalysators an Ruthenium bevorzugt zwischen 0,1 und 10 Gew.-% und der Anteil an Kobalt bevorzugt zwischen 0,1 und 50 Gew.-%, jeweils bezogen auf das Gesamtgewicht der Aktivmasse des Katalysators.

Zur Herstellung der Katalysatoren mit der genannten Zusammensetzung sind verschiedene Verfahrensweisen möglich; sie sind beispielsweise durch dem Fachmann bekannte Fällungsverfahren und bevorzugt durch Tränkungsverfahren erhältlich.

Hierbei ist es wesentlich, dass der Katalysator in Form kleiner Katalysatorformkörper eingesetzt wird. Mit kleinen Katalysatorformkörpern sind solche gemeint, deren Durchmesser bei Kugel- oder Strangform jeweils einen Durchmesser von < 3 mm, bei Tablettenform eine Höhe von < 3 mm und bei allen anderen Geometrien jeweils einen Äquivalentdurchmesser $L = 1/a'$ von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorformkörpers ($mm_s^2$) und $V_p$ das Volumen des Katalysatorformkörpers ($mm_p^3$) ist.

Besonders bevorzugt sind Katalysatorformkörper, die bei Kugel- oder Strangform jeweils einen Durchmesser von < 2,5 mm, bei Tablettenform eine Höhe von < 2,5 mm und bei allen anderen Geometrien jeweils einen Äquivalentdurchmesser L=1/a' von < 0,65 mm aufweisen.

Im erfindungsgemäßen Verfahren sind durch die kleine spezifische Dimension der Katalysatorpartikel die Diffusionswege der Reaktanden und auch der Produkte geringer. Die mittlere Verweilzeit der Moleküle in den Poren und die Wahrscheinlichkeit einer unerwünschten Folgereaktion werden hierdurch herabgesetzt. Als Folge der definierten Verweilzeit kann damit eine erhöhte Selektivität, insbesondere in Richtung des erwünschten EDAs und DETAs, erreicht werden.

Bevorzugt wird die erste Verfahrensstufe in einem ersten Reaktorbereich oder einem ersten Reaktor durchgeführt und die zweite Verfahrensstufe in einem zweiten Reaktorbereich oder einem zweiten Reaktor. Hierbei liegt in jedem Reaktorbereich oder Reaktor der Katalysator jeweils bevorzugt als Festbett vor. Die erste und die zweite Verfahrensstufe können beispielsweise jeweils in einem getrennten Rohrreaktor oder Rohrbündelreaktor durchgeführt werden, oder aber auch in jeweils einem Bereich eines Rohrreaktors oder Rohrbündelreaktors, bevorzugt jeweils im geraden Durchgang.

Das Katalysatorbett wird bevorzugt sowohl am Eingang als am Ausgang des Reaktors von einem Inertmaterial umgeben. Als Inertmaterial können zum Beispiel Pallringe, Kugeln aus einem inerten Material (zum Beispiel Keramik, Steatit, Aluminium) eingesetzt werden.

Der Reaktor kann sowohl in Sumpf- als in Rieselfahrweise betrieben werden. Bei der bevorzugten Rieselfahrweise wird bevorzugt ein Flüssigkeitsverteiler für den Reaktorfeed am Eingang des Reaktors eingesetzt. Wenn zwei Reaktoren eingesetzt werden, können beide in Sumpf- oder Rieselfahrweise betrieben werden. Alternativ kann der erste Reaktor in Sumpf- und der zweite Reaktor in Rieselfahrweise betrieben werden, oder umgekehrt.

Vorteilhaft sind der erste und der zweite Reaktorbereich durch eine Inertmaterialschicht voneinander getrennt, die insbesondere eine Dicke im Bereich von 20 cm bis 200 cm, bevorzugt 30 bis 70 cm, besonders bevorzugt von 30 cm, aufweist.

Zur Aufrechterhaltung der Katalysatoraktivität werden bevorzugt 0,01 - 2,00 Gew.-%, besonders bevorzugt 0,20 - 0,60 Gew.-%, Wasserstoff (bezogen auf den Reaktorfeed MEG + $NH_3$) in den Reaktor gefahren.

Im bevorzugt kontinuierlichen Betrieb werden bei einer Katalysatorbelastung WHSV (weight hourly space velocity) von 0,05 - 1,25 kg/kg*h, bevorzugt 0,1 - 0,4 kg/kg*h (kg MEG pro kg Kat. pro Stunde) im Umsatzbereich von 40 - 50 %, bezogen auf MEG, eine Selektivität für MEOA zwischen 10 % und 50 % und für die Summe aus EDA und DETA zwischen 30 % und 90 %, erreicht.

Als weitere Produkte fallen im erfindungsgemäßen Verfahren geringe Mengen an Diethanolamin, DEOA ($S_{DEOA}$, im Allgemeinen 0 - 5 Gew.-%), Triethanolamin, TEOA ($S_{TEOA}$, im Allgemeinen 0 - 2 Gew.-%) und höhere Amine ($S_{höhere}$ amine, im Allgemeinen 2 - 12 Gew.-%) an.

Im Allgemeinen enthalten die Reaktionsrohprodukte des erfindungsgemäßen Verfahrens nur geringe Mengen an zyklischen Aminen als Reaktionsprodukte (in der Regel in Mengen < 20 Gew.-%, insbesondere < 15 Gew.-%, ganz besonders 6 bis 12 Gew.-%).

Im Allgemeinen enthalten die Reaktionsrohprodukte des erfindungsgemäßen Verfahrens nur geringe Mengen an tertiären Aminen als Reaktionsnebenprodukte (in der Regel in Mengen < 10 Gew.-%, insbesondere < 7 Gew.-%, ganz besonders 0 bis 4 Gew.-%.

Die Aufarbeitung der im erfindungsgemäßen Verfahren anfallenden Produktströme, die vor allem das besonders gewünschte EDA und DETA, aber auch Aminoethylethanolamin (AEEA), Triethylentetramin (TETA), Piperazin (PIP), N-(2-Aminoethyl)-piperazin (AE-PIP) MEOA und unumgesetztes MEG enthalten, kann nach dem Fachmann bekannten Destillationsverfahren erfolgen. (Vergl. z. B. PEP Report No. 138, "Alkyl Amines", SRI International, 03/1981, Seiten 81-99, 117, und DE-A-10349059 (BASF-AG)).

Die zur destillativen Reingewinnung der einzelnen Produkte, vor allem des besonders gewünschten EDAs und DETAs, benötigten Destillationskolonnen können durch den Fachmann mit ihm geläufigen Methoden ausgelegt werden (z. B. Zahl der Trennstufen, Rücklaufverhältnis, etc.).

Die Auftrennung des aus der Umsetzung resultierenden Reaktionsaustrags erfolgt insbesondere durch mehrstufige Destillation.

Zum Beispiel erfolgt die Auftrennung des aus der Umsetzung resultierenden Reaktionsaustrags in zwei Trennsequenzen durch mehrstufige Destillation, wobei in der ersten Trennsequenz zunächst Ammoniak und gegebenenfalls vorhandener Wasserstoff abgetrennt werden und in der zweiten Trennsequenz eine Auftrennung in unumgesetztes MEG sowie MEOA, EDA, PIP, DETA, AEEA, AE-PIP, TETA und höhere Ethylenamine erfolgt.

Bei der Auftrennung des aus der Umsetzung resultierenden Reaktionsaustrags anfallender Ammoniak wird bevorzugt in die Umsetzung zurückgeführt.

Bevorzugt kann die Aminierung in Gegenwart von Wasser durchgeführt werden; die Zugabe von Wasser führt zu besseren Selektivitäten für MEOA.

Vorteilhaft Monoethanolamin aus dem Produktgemisch der hydrierenden Aminierung von Monoethylenglykol in die Umsetzung rezykliert werden, insbesondere in den zweiten Reaktorbereich oder den zweiten Reaktor.

Die Erfindung wird im Folgenden anhand eines Ausführungsbeispiels näher erläutert:

In einen ersten Reaktor wurden 83 g eines Standardkatalysators gefüllt in Form von 5 x 3 mm Tabletten, enthaltend, jeweils in Gewichtsprozenten: Nickeloxid 49,5, Kupferoxid 16,5, Zirkoniumdioxid 29,5, Molybdänoxid 1,5 und Graphit 3 und in einen zweiten, in Serie geschalteten Reaktor, 30 g eines wie nachstehend beschrieben hergestellten Katalysators:

151 g $Al_2O_3$-Stränge mit einem Durchmesser von 1,5 mm wurden mit 128 ml einer wässrigen Tränklösung, die 148 g Ru-Nitrosylnitrat und 46,5 g Kobaltnitrat-6-Hydrat enthielt, unter mehrmaligem guten Durchmischen bei Raumtemperatur für 60 Minuten stehen gelassen. Anschließend wurde für 4 Stunden bei 120°C unbewegt getrocknet und für 2 Stunden bei 600°C mit 150 L/h Luft calciniert. Der Katalysator enthielt 4,1 Gew.-% metallisches Kobalt und 7,1 Gew.-% metallisches Ruthenium, jeweils bezogen auf das Gesamtgewicht des Katalysators.

Nach Aktivierung mit Wasserstoff wurden 14,8/h MEG, 42,4 g/h Ammoniak und 14,6 Nl/h Wasserstoff bei einem Druck von 200 bar absolut kontinuierlich dem ersten Reaktor zugeführt. Die Temperatur im ersten Reaktor wurde isotherm auf 150°C eingestellt, im zweiten Reaktor auf 170°C. Der gaschromatographisch bestimmte MEG-Umsatz betrug 42,6 %, wobei die in der nachfolgenden Tabelle aufgeführten Anteile der Komponenten EDA, ME-OA, DEOA, PIP, DETA und AEEA erhalten wurden.

| Beispiel | Temperatur (°C) | Druck (bar) | $H_2$(mol $H_2$/mol MEG) | Belastung (kg/l*h) | MV ($NH_3$/MEG) | Umsatz MEG % |
|---|---|---|---|---|---|---|
| | 150*-170** | 200 | 0,6 | 0,10 | 15 | 42,6 |
| * in der 1. Verfahrensstufe<br>** in der 2. Verfahrensstufe | | | | | | |

| Beispiel | EDA % | MEOA % | PIP % | DETA % | AEEA % | DEOA % | Rest % | EDA/ PIP |
|---|---|---|---|---|---|---|---|---|
| | 49,5 | 29,0 | 6,8 | 3,9 | 5,7 | 1,7 | 3,4 | 7,3 |

**Patentansprüche**

1. Verfahren zur Herstellung von Ethylenaminen und Ethanolaminen durch hydrierende Aminierung von Monoethylenglykol und Ammoniak in Gegenwart eines Katalysators, **dadurch gekennzeichnet, dass** das Verfahren in zwei Verfahrensstufen durchgeführt wird, wobei

- in der ersten Verfahrensstufe die Aminierung an einem Hydroaminierungskatalysator bis zu einem Monoethylenglykol-Umsatz von maximal 40 % durchgeführt wird und
- in der zweiten Verfahrensstufe ein geträgerter Katalysator mit einer Aktivmasse enthaltend Ruthenium und Kobalt und daneben kein weiteres Metall der Gruppe VIII sowie kein Metall der Gruppe IB, eingesetzt wird, in Form von Katalysatorformkörpern, die bei Kugelform oder Strangform jeweils einen Durchmesser von < 3 mm, bei Tablettenform eine Höhe von < 3 mm und bei allen anderen Geometrien jeweils einen Äquivalentdurchmesser L = 1/a' von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorformkörpers ($mm_s^2$) und $V_p$ das Volumen des Katalysatorformkörpers ($mm_p^3$) ist, eingesetzt wird und die zweite Verfahrensstufe bei einer um mindestens 10 °C höheren Temperatur als die erste Verfahrensstufe durchgeführt wird.

2.   Verfahren nach Anspruch 1, **dadurch gekennzeichnet, dass** in der ersten Verfahrensstufe die Aminierung bis zu einem Monoethylenglykol-Umsatz von maximal 30 % durchgeführt wird.

3.   Verfahren nach Anspruch 1 oder 2, **dadurch gekennzeichnet, dass** der in der zweiten Verfahrensstufe eingesetzte Katalysator einen Anteil von 0,1 bis 10 Gew.-% an Ruthenium und einen Anteil von 0,1 bis 50 Gew.-% an Kobalt, jeweils bezogen auf das Gesamtgewicht des Katalysators, enthält.

4.   Verfahren nach einem der Ansprüche 1 bis 3, **dadurch gekennzeichnet, dass** der Katalysatorträger für den in der zweiten Verfahrensstufe eingesetzten Katalysator γ-Aluminiumoxid enthält.

5.   Verfahren nach einem der Ansprüche 1 bis 4, **dadurch gekennzeichnet, dass** der in der zweiten Verfahrensstufe eingesetzte geträgerte Katalysator in Form von Katalysatorformkörpern eingesetzt wird, die bei Kugel- oder Strangform jeweils einen Durchmesser von < 2,5 mm, bei Tablettenform eine Höhe von < 2,5 mm und bei allen anderen Geometrien jeweils einen Äquivalentdurchmesser L=1/a' von < 0,65 mm, aufweisen.

6.   Verfahren nach einem der Ansprüche 1 bis 5, **dadurch gekennzeichnet, dass** die erste Verfahrensstufe in einem ersten Reaktorbereich oder einem ersten Reaktor und die zweite Verfahrensstufe in einem zweiten Reaktorbereich oder einem zweiten Reaktor durchgeführt wird.

7.   Verfahren nach Anspruch 6, **dadurch gekennzeichnet, dass** der erste und der zweite Reaktorbereich durch eine Inertmaterialschicht voneinander getrennt sind.

8.   Verfahren nach Anspruch 7, **dadurch gekennzeichnet, dass** die Dicke der Inertmaterialschicht 20 cm bis 200 cm, bevorzugt 30 bis 70 cm, besonders bevorzugt 30 cm, beträgt.

9.   Verfahren nach einem der Ansprüche 1 bis 8, **dadurch gekennzeichnet, dass** Monoethanolamin aus dem Produktgemisch der hydrierenden Aminierung von Monoethylenglykol in die Umsetzung rezykliert wird.

10.  Verfahren nach Anspruch 9, **dadurch gekennzeichnet, dass** Monoethanolamin aus dem Produktgemisch der hydrierenden Aminierung in den zweiten Reaktorbereich oder in den zweiten Reaktor rezykliert wird.

**Claims**

1.   A process for preparing ethylene amines and ethanolamines by hydrogenative amination of monoethylene glycol and ammonia in the presence of a catalyst, wherein the process is carried out in two process stages in which

- in the first process stage, the amination is carried out over a hydroamination catalyst to a monoethylene glycol conversion of not more than 40% and
- in the second process stage, a supported catalyst having an active composition comprising ruthenium and cobalt and no further additional metal of group VIII and also no metal of group IB is used in the form of shaped catalyst bodies which in the case of a spherical shape or rod shape in each case have a diameter of < 3 mm, in the case of a pellet shape have a height of < 3 mm and in the case of all other geometries in each case have an equivalent diameter L = 1/a' of < 0.70 mm, where a' is the external surface area per unit volume ($mm_s^2/mm_p^3$) and:

$$a' = \frac{Ap}{Vp}$$

where Ap is the external surface area of the shaped catalyst body ($mm_s^2$) and $V_p$ is the volume of the shaped catalyst body ($mm_p^3$), and the second process stage is carried out at a temperature which is at least 10°C higher than that in the first process stage.

**2.** The process according to claim 1, wherein the amination is carried out to a monoethylene glycol conversion of not more than 30% in the first process stage.

**3.** The process according to claim 1 or 2, wherein the catalyst used in the second process stage comprises a proportion of from 0.1 to 10% by weight of ruthenium and a proportion of from 0.1 to 50% by weight of cobalt, in each case based on the total weight of the catalyst.

**4.** The process according to any of claims 1 to 3, wherein the catalyst support for the catalyst used in the second process stage comprises γ-aluminum oxide.

**5.** The process according to any of claims 1 to 4, wherein the supported catalyst used in the second process stage is used in the form of shaped catalyst bodies which in the case of a spherical or rod shape in each case have a diameter of < 2.5 mm, in the case of a pellet shape have a height of < 2.5 mm and in the case of all other geometries in each case have an equivalent diameter L = 1/a' of < 0.65 mm.

**6.** The process according to any of claims 1 to 5, wherein the first process stage is carried out in a first reactor region or a first reactor and the second process stage is carried out in a second reactor region or a second reactor.

**7.** The process according to claim 6, wherein the first reactor region and the second reactor region are separated from one another by a bed of inert material.

**8.** The process according to claim 7, wherein the thickness of the bed of inert material is from 20 cm to 200 cm, preferably from 30 to 70 cm, particularly preferably 30 cm.

**9.** The process according to any of claims 1 to 8, wherein monoethanolamine from the product mixture of the hydrogenative amination of monoethylene glycol is recycled to the reaction.

**10.** The process according to claim 9, wherein monoethanolamine from the product mixture of the hydrogenative amination is recycled to the second reactor region or to the second reactor.

**Revendications**

**1.** Procédé pour la préparation d'éthylène-amines et d'éthanolamines par amination avec hydrogénation de monoéthylèneglycol et d'ammoniac en présence d'un catalyseur, **caractérisé en ce que** le procédé est réalisé en deux étapes de procédé

- l'amination étant réalisée, dans une première étape de procédé, sur un catalyseur d'hydroamination jusqu'à une conversion du monoéthylèneglycol d'au maximum 40% et
- un catalyseur supporté présentant une masse active contenant du ruthénium et du cobalt et pour le reste aucun métal du groupe VIII ni aucun métal du groupe IB n'étant utilisé dans la deuxième étape de procédé, sous forme de corps façonnés catalytiques, qui présentent, dans le cas d'une forme sphérique ou de brin, à chaque fois un diamètre < 3 mm, dans le cas d'une forme de comprimés une hauteur < 3 mm et dans le cas de toutes les autres géométries à chaque fois un diamètre équivalent L = 1/a' < 0,70 mm, a' représentant la surface externe par unité de volume ($mm_s^2/mm_p^3$) :

$$a' = \frac{A_p}{V_p}$$

$A_p$ étant la surface externe du corps façonné catalytique ($mm_s^2$) et $V_p$ le volume du corps façonné catalytique ($mm_p^3$), et la deuxième étape de procédé étant réalisée à une température supérieure d'au moins 10°C à celle de la première étape de procédé.

**2.** Procédé selon la revendication 1, **caractérisé en ce que**, dans la première étape de procédé, l'amination est réalisée jusqu'à une conversion du monoéthylèneglycol d'au maximum 30%.

**3.** Procédé selon la revendication 1 ou 2, **caractérisé en ce que** le catalyseur utilisé dans la deuxième étape de procédé contient une proportion de 0,1 à 10% en poids de ruthénium et une proportion de 0,1 à 50% en poids de cobalt, à chaque fois par rapport au poids total du catalyseur.

**4.** Procédé selon l'une quelconque des revendications 1 à 3, **caractérisé en ce que** le support de catalyseur pour le catalyseur utilisé dans la deuxième étape de procédé contient du $\gamma$-oxyde d'aluminium.

**5.** Procédé selon l'une quelconque des revendications 1 à 4, **caractérisé en ce que** le catalyseur supporté utilisé dans la deuxième étape de procédé est utilisé sous forme de corps façonnés catalytiques, qui présentent, dans le cas d'une forme sphérique ou de brin, à chaque fois un diamètre < 2,5 mm, dans le cas d'une forme de comprimés une hauteur < 2,5 mm et dans le cas de toutes les autres géométries à chaque fois un diamètre équivalent $L=1/a'$ < 0,65 mm.

**6.** Procédé selon l'une quelconque des revendications 1 à 5, **caractérisé en ce que** la première étape de procédé est réalisée dans une première zone de réacteur ou dans un premier réacteur et la deuxième étape de procédé est réalisée dans une deuxième zone de réacteur ou dans un deuxième réacteur.

**7.** Procédé selon la revendication 6, **caractérisé en ce que** la première et la deuxième zone de réacteur sont séparées l'une de l'autre par une couche de matériau inerte.

**8.** Procédé selon la revendication 7, **caractérisé en ce que** l'épaisseur de la couche de matériau inerte est de 20 cm à 200 cm, de préférence de 30 à 70 cm, de manière particulièrement préférée de 30 cm.

**9.** Procédé selon l'une quelconque des revendications 1 à 8, **caractérisé en ce que** la monoéthanolamine du mélange de produits de l'amination avec hydrogénation du monoéthylèneglycol est recyclée dans la transformation.

**10.** Procédé selon la revendication 9, **caractérisé en ce que** la monoéthanolamine du mélange de produits de l'amination avec hydrogénation est recyclée dans la deuxième zone du réacteur ou dans le deuxième réacteur.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4014933 A
- WO 05014623 A
- DE 102005019373
- DE 102005047464
- WO 9638226 A
- US 4855505 A
- EP 0839575 A
- DE 10349059 A

**In der Beschreibung aufgeführte Nicht-Patentliteratur**

- Alkyl Amines. *SRI International, 03/1981,* 7, 8 13-1643-107113, 117
- **C.M. BARNES.** *Ind. Eng. Chem. Prod. Res. Dev.,* 1981, vol. 20, 399-407
- *Journal of Catalysis,* 1996, vol. 161, 96-106
- Alkyl Amines. *SRI International, 03/1981,* 81-99117